# EUROPÄISCHE PATENTSCHRIFT

(11) **EP 0 821 977 B1**
(45) Veröffentlichungstag und Bekanntmachung des Hinweises auf die Patenterteilung: **25.06.2003**
(21) Anmeldenummer: 97112920.0
(22) Anmeldetag: 28.07.1997
(51) Int. Cl.: A61M 16/00, A61B 5/085

(54) **Vorrichtung zur Steuerung eines Beatmungsgerätes zur Therapie der Schlafapnoe**
Ventilation apparatus for the treatment of sleep apnoea
Appareil de ventilation artificielle pour le traitement de l'apnée du sommeil

(30) Priorität: 30.07.1996 DE 29613169 U
(43) Veröffentlichungstag der Anmeldung: 04.02.1998
(73) Patentinhaber: Gottlieb Weinmann Geräte für Medizin und Arbeitsschutz GmbH + Co., 22525 Hamburg (DE)
(72) Erfinder: Graetz, Bernd, D-22869 Schenefeld (DE); Maurer, Jörg, D-22113 Oststeinbek (DE)
(74) Vertreter: Liebelt, Rolf, Dipl.-Ing.

(56) Entgegenhaltungen:
- EP-A- 0 705 615
- DE-A- 4 429 561
- US-A- 3 598 111
- US-A- 5 245 995

## Beschreibung

Die Erfindung betrifft ein Beatmungsgerät zur Therapie der Schlafapnoe nach dem Oberbegriff des Anspruches 1.

Eine nicht unbeachtliche Anzahl von Menschen leidet unter Schlafstörungen, die sich auf das Tagesbefinden dieser Menschen auswirken und deren soziale und berufliche Leistungsfähigkeit sowie deren Lebensqualität zum Teil erheblich beeinträchtigen. Eine dieser Schlafstörungen ist die obstruktive Schlafapnoe, bei der durch eine Reduktion des Muskeltonus' die oberen Atemwege ganz oder teilweise kollabieren und die vorrangig mit der sogenannten CPAP-Therapie (CPAP = Continuous Positive Airway Pressure) behandelt wird, indem dem Patienten während des Schlafens ein Luftstrom aus einem atemfähigen Gas über eine Nasalmaske zugeführt wird. Die Maske ist über einen Schlauch mit einem Beatmungsgerät verbunden, das einen Lüfter aufweist, der einen Gasstrom mit einem Überdruck, der zwischen 5 und 20 mbar einstellbar ist, erzeugt. Der Gasstrom wird dem Patienten entweder mit konstantem Druck zugeführte oder zur Erleichterung der Atemarbeit des Patienten beim Ausatmen auf ein niedrigeres Druckniveau abgesenkt. Obwohl Schlafapnoephasen nur kurzfristig auftreten und einen geringen zeitlichen Anteil des Schlafes ausmachen, läuft der Lüfter bei beiden Verfahren im Dauerbetrieb, d. h. während der gesamten Schlafdauer (Nacht), was die Akzeptanz dieser Schlafapnoe-Behandlung deutlich vermindert. Zur Beseitigung dieser Nachteile sind aus der EP-A 0 705 615 ein Verfahren zur Steuerung eines Beatmungsgerätes sowie ein Beatmungsgerät zur Durchführung des Verfahrens bekannt, bei dem mit der Oszilloresistometrie (oszillatorische Resistenz-Messung = ORM) Änderungen im Phasenwinkel der oszillatorischen Druckamplitude, die dem Atemwiderstand des Patienten proportional ist, kontinuierlich gemessen werden, wobei nach dem Bestimmen des individuellen Atemwiderstandswertes (Basiswert der Druckamplitude) bei Abweichungen von diesem Wert dem Patienten Atemgas unter Druck zugeführt und die Gaszufuhr beendet wird, sobald der Basiswert wieder oder annähernd erreicht ist. Die Dynamik des Verfahrens wird von der Zeit bestimmt, die von der Erkennung der Widerstandszunahme (Erhöhung der Druckamplitude) bis zum Erreichen des therapeutisch wirksamen CPAP-Druckes vergeht. Innerhalb dieses Zeitfensters, d. h. noch vor dem Erreichen des therapeutisch wirksamen CPAP-Druckes, können noch Apnoen auftreten.

Aufgabe der Erfindung ist es nun, die Sensibilität der Steuerung eines Beatmungsgerätes durch Verkürzen der Ansprechzeit zu verbessern.

Diese Aufgabe wird mit einem Beatmungsgerät zur Therapie der Schlafapnoe der eingangs beschriebenen Art dadurch gelöst, daß als Phasensprung auftretende signifikante Veränderungen des Phasenwinkels vorzugsweise mit der FFT (Fast Fourier Transformation), der Autokorrelation, der Cluster-Analyse oder der Median-Analyse aus dem Signal des Phasenwinkels gefiltert werden sowie daraus ein Steuersignal zur Anpassung des Atemdruckes generiert wird.

Das erfindungsgemäße Beatmungsgerät zur Therapie von Schlafapnoe-Patienten wird schon dann aktiviert, d. h. es wird dem Patienten schon dann Atemgas zugeführt, wenn durch eine sich bildende Apnoe die Atemtätigkeit des Patienten gestört ist, was zu als Phasensprung auftretenden signifikanten Veränderungen des Phasenwinkels führt, bevor Auswirkungen auf den Atemwiderstand feststellbar sind. Eine Störung der Atemtätigkeit des Patienten ist von einer Veränderung des Atemdruckes und Atemflusses des Patienten begleitet. Dieser Druck und Fluß werden, ohne das Befinden des Patienten zu beeinflussen, auf einfache Weise zuverlässig und reproduzierbar mit der oszillatorischen Druckamplitude bestimmt und daraus der Phasenwinkel ermittelt. Markante Veränderungen des Phasenwinkels, z. B. ein Phasensprung durch Veränderung der Compliance der Atemwege, treten schon in einem signifikanten Zeitraum, bevor Änderungen des Atemdruckes und Atemflusses erfaßt werden können, auf und bilden die Steuergröße für ein frühzeitiges Ein- und Ausschalten des Therapiegerätes, so daß eine Unterversorgung des Patienten mit Sauerstoff nicht eintritt und eine Entlastung des Patienten erzielt wird, da die Druckanpassung des Atemgases sehr zeitig einsetzt und damit ein "weicher Übergang" zu dem jeweils erforderlichen Atemgasdruck erfolgen kann.

Bei einer beispielhaften Ausführungsform des Beatmungsgerätes nach der Erfindung ist eine Einrichtung vorhanden, mit der dem Atemfluß eines Patienten ein sinusförmiger Wechselfluß überlagert wird, der mit einer ventillosen Membranpumpe erzeugt werden kann. Zur Bestimmung der oszillatorischen Druckamplitude, die dem Atemwiderstand des Patienten entspricht, wird der zeitliche Druckverlauf in der Nasalmaske, über welche dem Patienten Atemgas, z. B. vom Gebläse eines Beatmungsgerätes, zugeführt wird, gemessen sowie der Anteil der überlagerten Schwingungen herausgefiltert. Gleichzeitig wird der Druckverlauf in der Membranpumpe erfaßt. Aus der zeitlichen Differenz dieser beiden Drucksignale wird der Phasenwinkel ermittelt, dessen Verlauf vor dem Beginn einer Apnoe markante Veränderungen wie Phasensprünge aufweist, die vermutlich durch Veränderungen der Atemwegs-Compliance verursacht werden. Diese vor dem Beginn einer Apnoe oder Atemunregelmäßigkeit auftretenden signifikanten Änderungen werden z. B. mit der FFT (Fast Fourier Transformation), der Autokorrelation, der Cluster- oder Median-Analyse detektiert und lösen erfindungsgemäß, z. B. durch Erhöhen der Drehzahl des Gebläses des Beatmungsgerätes, einen Druckanstieg im Strom des zugeführten Atemgases aus. Dabei wird mit dem oszillatorischen Drucksignal, das dem Atemwiderstand des Patienten proportional ist, nicht nur der Druckanstieg überprüft, sondern auch der Druck des Atemgases geregelt.

Ein Beatmungsgerät nach der Erfindung besteht aus einer mit einer Atemmaske verbundenen und zweckmäßigerweise als Lüfter ausgebildeten Druckgasquelle und einer Einrichtung zum kontinuierlichen Messen des Phasenwinkels (der zeitlichen Differenz zwischen Atemfluß und Atemdruck) und der dem Atemwiderstand entsprechenden Druckamplitude sowie zum Bestimmen des individuellen Atemwiderstandswertes eines Patienten nach dem ORM-Prinzip und ist durch eine Steuer-Regeleinrichtung gekennzeichnet, die mit aus dem Signal des Phasenwinkels gefilterten als Phasensprung auftretenden signifikanten Veränderungen des Phasenwinkels die Druckgasquelle so aktiviert bzw. steuert, daß dem Patienten Atemgas mit optimalen, d. h. dem therapeutisch wirksamen Druck zugeführt wird.

Bei einer zweckmäßigen Weiterbildung der Erfindung kann das Beatmungsgerät noch mit einem auf die Bedürfnisse des Patienten einstellbaren Druckregler für das Atemgas versehen sein, wodurch die Akzeptanz des Therapiegerätes gesteigert wird, da der Patient mit einem für ihn angenehmen konstanten Druck beatmet wird, wenn die oszillatorische Druckamplitude und/oder der Phasenwinkel der Druckamplitude und/oder der Atemfluß des Patienten dem (den) Basiswert(en) entspricht (entsprechen).

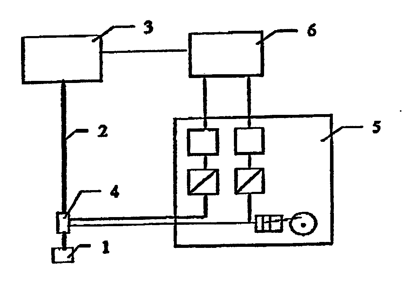

Ein Ausführungsbeispiel des erfindungsgemäßen Beatmungsgerätes wird noch an Hand der Zeichnung, in der es schematisch dargestellt ist, beschrieben.

Das Beatmungsgerät zur Therapie der Schlafapnoe weist eine auf der Nase eines Patienten anordenbare Atemmaske 1 auf, die über einen Atemschlauch 2 mit einer als Lüfter ausgebildeten Druckgasquelle 3 verbunden ist. Im Atemschlauch 2 sind vor der Atemmaske 1 die Sensoren 4 einer Einrichtung 5 zum Erzeugen, Messen und Filtern der oszillatorischen Druckamplitude nach dem ORM-Prinzip sowie des Phasenwinkels vorgesehen. Von einer Steuer-Regeleinrichtung 6, die mit der Einrichtung 5 verbunden und auf den individuellen Basiswert der dem Atemwiderstand des Patienten entsprechenden oszillatorischen Druckamplitude einstellbar ist, wird die Druckgasquelle 3 so aktiviert, daß dem Patienten Atemgas bei signifikanten Veränderungen des Phasenwinkels zugeführt wird. Die Einrichtung 5 umfaßt auch eine ventillose Membranpumpe (nicht gezeigt), mit der dem Atemfluß ein sinusförmiger Wechselfluß überlagert wird.

## Patentansprüche

1. Beatmungsgerät zur Therapie der Schlafapnoe bestehend aus einer mit einer Atemmaske verbundenen Druckgasquelle und Einrichtungen
a) zum kontinuierlichen Messen der zeitlichen Differenz zwischen Atemfluß und Atemdruck als Phasenwinkel und der dem Atemwiderstand entsprechenden Druckamplitude,
b) zum Bestimmen des individuellen Atemwiderstandswertes als Basiswert der Druckamplitude eines Patienten mit Hilfe der Oszilloresistometrie,
und
c) zum Regeln des Atemgasdruckes in Abhängigkeit vom Phasenwinkel und der Druckamplitude, **gekennzeichnet durch**
d) Einrichtungen zum Erfassen von als Phasensprung auftretenden signifikanten Veränderungen des Phasenwinkels **durch** Herausfiltern aus dem Signal des Phasenwinkels sowie zum Generieren eines Steuersignales zur Anpassung des Atemgasdruckes aus diesen signifikanten Veränderungen.

2. Beatmungsgerät nach Anspruch 1, **gekennzeichnet, durch** Einrichtungen
a) zum Erzeugen eines sinusförmigen Wechselflusses, der dem Atemfluß des Patienten überlagert wird,
und
b) zum gleichzeitigen Messen des zeitlichen Druckverlaufes im dem Patienten zugeführten Atemgasstrom sowie im überlagerten Wechselfluß zum Bestimmen der dem Atemwiderstand des Patienten entsprechenden oszillatorischen Druckgasamplitude und zum Ermitteln des Phasenwinkels **durch** Herausfiltern der überlagerten Schwingungen.

3. Beatmungsgerät nach Anspruch 1, **gekennzeichnet durch** eine ventillose Membranpumpe, mit der dem Atemfluß des Patienten ein sinusförmiger Wechselfluß überlagert wird.

4. Beatmungsgerät nach einem der vorhergehenden Ansprüche, **dadurch gekennzeichnet, daß** die Druckgasquelle (3) ein Lüfter ist.

5. Beatmungsgerät nach einem der Ansprüche 1 bis 4, **dadurch gekennzeichnet, daß** die als Phasensprung auftretenden signifikanten Veränderungen des Phasenwinkels mit der FFT (Fast Fourier Transformation) aus dem Signal des Phasenwinkels gefiltert werden.

6. Beatmungsgerät nach einem der Ansprüche 1 bis 4, **dadurch gekennzeichnet, daß** die als Phasensprung auftretenden signifikanten Veränderungen des Phasenwinkels mit dem Verfahren der Autokorrelation aus dem Signal des Phasenwinkels gefiltert werden.

7. Beatmungsgerät nach einem der Ansprüche 1 bis 4, **dadurch gekennzeichnet, daß** die als Phasensprung auftretenden signifikanten Veränderungen des Phasenwinkels mit dem Verfahren der Cluster-Analyse aus dem Signal des Phasenwinkels gefiltert werden.

8. Beatmungsgerät nach einem der Ansprüche 1 bis 4, **dadurch gekennzeichnet, daß** die als Phasensprung auftretenden signifikanten Veränderungen des Phasenwinkels mit dem kombinierten Verfahren der Autokorrelationsanalyse und der Mediananalyse aus dem Signal des Phasenwinkels gefiltert werden.

9. Beatmungsgerät nach einem der vorhergehenden Ansprüche, **gekennzeichnet durch** einen auf die Bedürfnisse des Patienten einstellbaren Druckregler für das Atemgas.

## Claims

1. Respirator for therapeutic treatment of sleep apnea consisting of a compressed gas source connected to a respiratory mask and devices
a) for continuously measuring the time difference between respiratory flow and respiratory pressure as a phase angle and the pressure amplitude corresponding to the respiratory resistance,
b) for determining by oscilloresistometry the individual respiratory resistance value of the patient as a basic value of the pressure amplitude,
and
c) for controlling the respiratory gas pressure as a function of the phase angle and the pressure amplitude,
**characterised by**
d) devices for detecting significant changes in the phase angle occurring as a phase jump by filtering from the phase angle signal and for generating a control signal from these significant changes for adapting the respiratory gas pressure.

2. Respirator according to claim 1, **characterised by** devices
a) for generating a sinusoidal alternating flux which is superimposed on the respiratory flow of the patient,
and
b) for simultaneous measuring of the pressure course over time in the respiratory gas flow supplied to the patient and in the superimposed alternating flux for determining the oscillatory compressed gas amplitude corresponding to the respiratory resistance of the patient and for ascertaining the and phase angle by filtering out the superimposed oscillations.

3. Respirator according to claim 1, **characterised by** a valve-free diaphragrn pump for superimposing a sinusoidal alternating flux onto the respiratory flow of the patient.

4. Respirator according to any of the preceding claims, **characterised in that** the compressed gas source (3) is a fan.

5. Respirator according to any of claims 1 to 4, **characterised in that** the significant changes of the phase angle occurring as a phase jump are filtered from the phase angle signal using FFT (Fast Fourier Transformation).

6. Respirator according to any of claims 1 to 4, **characterised in that** the significant changes of the phase angle occurring as a phase jump are filtered from the phase angle signal using the auto-correlation method.

7. Respirator according to any of claims 1 to 4, **characterised in that** the significant changes in the phase angle occurring as a phase jump are filtered from the phase angle signal using the cluster analysis method.

8. Respirator according to any of claims 1 to 4, **characterised in that** the significant changes in the phase angle occurring as a phase jump are filtered from the phase angle signal using the combined methods of auto-correlation analysis and median analysis.

9. Respirator according to any of the preceding claims, **characterised by** a pressure control member for the respiratory gas which can be adjusted to the patient's needs.

## Revendications

1. Appareil respiratoire destiné à soigner l'apnée du sommeil, se composant d'une source de gaz sous pression reliée à un masque respiratoire et de moyens pour :
a) mesurer en continu l'intervalle de temps entre le flux respiratoire et la pression respiratoire sous la forme d'un angle de phase, et l'amplitude de la pression correspondant à la résistance respiratoire,
b) déterminer la valeur individuelle de la résistance respiratoire sous la forme d'une valeur de base de l'amplitude de la pression chez un patient par oscillorésistométrie, et
c) réguler la pression du gaz respiratoire en fonction de l'angle de phase et de l'amplitude de la pression,
**caractérisé par**
d) des moyens pour mesurer des variations significatives de l'angle de phase, survenant sous la forme de saut de phase, en les extrayant par filtrage du signal de l'angle de phase, et à générer un signal de commande pour adapter la pression du gaz respiratoire à partir de ces variations significatives.

2. Appareil respiratoire selon la revendication 1, **caractérisé par** des moyens pour
a) générer un flux alternatif sinusoïdal qui est superposé au flux respiratoire du patient, et
b) mesurer simultanément la variation dans le temps de la pression dans le flux de gaz respiratoire amené au patient ainsi que dans le flux alternatif superposé, afin de déterminer l'amplitude oscillatoire du gaz sous pression correspondant à la résistance respiratoire du patient et de déterminer l'angle de phase en extrayant par filtrage les variations superposées.

3. Appareil respiratoire selon la revendication 1, **caractérisé par** une pompe à membrane sans clapet au moyen de laquelle un flux alternatif sinusoïdal est superposé au flux respiratoire du patient.

4. Appareil respiratoire selon l'une des revendications précédentes, **caractérisé en ce que** la source de gaz sous pression (3) est un ventilateur.

5. Appareil respiratoire selon l'une des revendications 1 à 4, **caractérisé en ce que** les variations significatives de l'angle de phase, survenant sous la forme de saut de phase, sont extraites du signal de l'angle de phase par filtrage au moyen de la FFT (Transformée de Fourier Rapide ou Fast Fourier Transformation pour les anglo-saxons).

6. Appareil respiratoire selon l'une des revendications 1 à 4, **caractérisé en ce que** les variations significatives de l'angle de phase, survenant sous la forme de saut de phase, sont extraites du signal de l'angle de phase par filtrage par le procédé de l'autocorrélation.

7. Appareil respiratoire selon l'une des revendications 1 à 4, **caractérisé en ce que** les variations significatives de l'angle de phase, survenant sous la forme de saut de phase, sont extraites du signal de l'angle de phase par filtrage par le procédé de l'analyse de nuées.

8. Appareil respiratoire selon l'une des revendications 1 à 4, **caractérisé en ce que** les variations significatives de l'angle de phase, survenant sous la forme de saut de phase, sont extraites du signal de l'angle de phase par filtrage par le procédé combiné de l'analyse par autocorrélation et de l'analyse de la médiane.

9. Appareil respiratoire selon l'une des revendications précédentes, **caractérisé par** un régulateur de la pression du gaz respiratoire, réglable en fonction des besoins du patient.
